# EUROPÄISCHE PATENTSCHRIFT

(11) **EP 0 798 286 B1**
(45) Veröffentlichungstag und Bekanntmachung des Hinweises auf die Patenterteilung: **25.07.2001**
(21) Anmeldenummer: 97250079.7
(22) Anmeldetag: 17.03.1997
(51) Int. Cl.: C07C 69/743, C07C 67/03, C07C 67/343, A61K 6/083, C08F 36/14

(54) **Multifunktionelle Vinylcyclopropan-Derivate**
Multifunctional vinylcyclopropane derivatives
Dérivés multifonctionnels du vinylcyclopropane

(30) Priorität: 18.03.1996 DE 19612004
(43) Veröffentlichungstag der Anmeldung: 01.10.1997
(73) Patentinhaber: Ivoclar Vivadent AG, 9494 Schaan (LI)
(72) Erfinder: Rheinberger, Volker, Dr, 9490 Vaduz (LI); Zeuner, Frank, Dr., 9490 Vaduz (LI); Moszner, Norbert, Prof.-Dr., 9492 Eschen (LI)
(74) Vertreter: UEXKÜLL & STOLBERG

(56) Entgegenhaltungen:
- Keine einschlägigen Dokumente gefunden

## Beschreibung

Die Erfindung betrifft multifunktionelle Vinylcyclopropan-Derivate, ein Verfahren zu deren Herstellung, deren Verwendung insbesondere als Dentalmaterial, ein diese enthaltendes Dentalmaterial sowie aus diesen erhältliche Polymere und Copolymere.

Substituierte Vinylcyclopropane sind aus dem Stand der Technik bekannt. So beschreiben T. Takahashi et al. in J. Polym. Sci., Part B, **3** (1965), 251, daß es bei der radikalischen Polymerisation von Vinylcyclopropanen zur Bildung 1,5-ringgeöffneter Verbindungen kommt, wobei radikalstabilisierende Substituenten in 1-Stellung die Ringöffnung begünstigen.

Weiter hat es sich gezeigt, daß 1,1-disubstituierte 2-Vinylcyclopropane, wie z.B. das flüssige 1,1-Bis(ethoxycarbonyl)- oder 1,1-Dicyano-2-vinylcyclopropan im Vergleich zu herkömmlichen Vinylmonomeren, wie Acrylnitril oder Methylmethacrylat, bei Polymerisation einen etwas geringeren Volumenschrumpf aufweisen. Allerdings wurde für speziell substituiertes Vinylcyclopropan, nämlich 1,1-Bis(phenoxycarbonyl)-2-vinylcyclopropan auch eine Volumenexpansion nach radikalischer Polymerisation festgestellt (vgl. J. Sugiyama et al., Macromolecules **27** (1994), 5543).

Bekannte ringöffnende Monomere, wie methylengruppenhaltige Spiroorthocarbonate, Spiroorthoester oder bicyclische Orthoester (vgl. R.K. Sadhir et al., Expanding Monomers, CRC Press, Boca Raton 1992) sind in der Regel feuchtigkeitsempfindlich, nur durch aufwendige Synthesen zugänglich und durch radikalische Polymerisation lediglich zu Polymeren mit geringer Molmasse verarbeitbar. Weiter führt die radikalische Polymerisation von Spiroorthocarbonaten oder Spiroorthoestern zu Polymeren mit Carbonat-Ether-Gruppen oder Ester-Ether-Gruppen in der Hauptkette. Diese Gruppen sind jedoch hydrolytisch oder enzymatisch einfach spaltbar, weshalb diese Polymere unter entsprechenden Bedingungen wenig stabil sind.

Bisher bekannte 1,1-disubstituierte 2-Vinylcyclopropane haben meist nur eine polymerisationsfähige Gruppe, so daß bei ihrer Polymerisation im Vergleich zu üblichen Vernetzermonomeren, z.B. auf Di(meth)acrylat-Basis, nur Polymere mit schlechteren mechanischen Eigenschaften hergestellt werden können. Vinylcyclopropane mit zwei polymerisationsfähigen Gruppen, wie z.B. 1-Vinyl-6,7-benzo-4,9-dioxaspiro[2.6]nonan (vgl. F. Sanda et al., Macromolecules **27** (1994), 1099) oder 1,10-Bis(vinyl)-4,8,12,15-tetraoxatrispiro[2.2.2.2.2]pentadecan (vgl. T. Okazaki et al., Macromolecules **28**, (1995) 6026), die cyclische Acetale mit Vinylcyclopropan-Gruppen darstellen, sind zwar bekannt, sie ergeben bei der radikalischen Polymerisation jedoch ebenfalls Polymere mit hydrolytisch oder enzymatisch spaltbaren Ester-Bindungen in der Hauptkette.

Der Erfindung liegt demgemäß die Aufgabe zugrunde, Vinylcyclopropane zur Verfügung zu stellen, die radikalisch polymerisierbar sind, aufgrund ihrer Multifunktionalität zu vernetzten Polymeren führen, Polymere bilden, die keine hydrolytisch spaltbaren Gruppen in der Hauptkette tragen, und sich insbesondere als Komponente von Dentalmaterialien einsetzen lassen.

Diese Aufgabe wird durch die multifunktionellen Vinylcyclopropan-Derivate nach den Ansprüchen 1 und 2 gelöst.

Gegenstand der vorliegenden Erfindung sind ebenfalls das Verfahren zur Herstellung der multifunktionellen Vinylcyclopropan-Derivate nach den Ansprüchen 3 und 4, deren Verwendung nach den Ansprüchen 5 und 7, ein Dentalmaterial mit Gehalt an den multifunktionellen Vinylcyclopropan-Derivaten nach den Ansprüchen 7 und 8 sowie Polymere und Copolymere der mehrfunktionellen Vinylcyclopropan-Derivate nach Anspruch 9.

Die erfindungsgemäßen multifunktionellen Vinylcyclopropan-Derivate sind Verbindungen der folgenden allgemeinen Formel (I), Stereoisomere davon und Gemische solcher Stereoisomeren 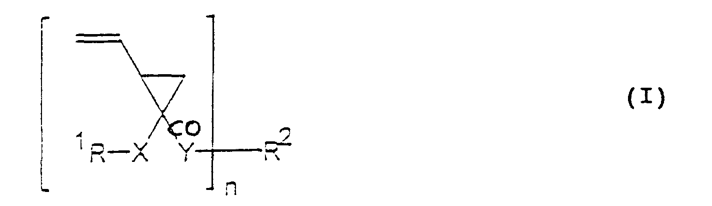 wobei R¹, R², R³, R⁴, X, Y und n unabhängig voneinander die folgenden Bedeutungen haben:
- R¹ =: H, ein- oder mehrfach mit OH, Halogen, C₁- bis C₆-Alkoxy oder COOH substituiertes oder unsubstituiertes C₁- bis C₁₂-Alkyl oder Cycloalkyl oder C₆- bis C₁₄-Aryl;
- R² =: n-fach substituiertes C₁- bis C₂₀-Alkylen oder - Cycloalkylen, das durch O, S, N oder NH unterbrochen sein kann, oder n-fach substituiertes C₆- bis C₁₄-Arylen;
- X =: CO, CO-O, CO-S, CO-NR³, SO₂ oder entfällt,
wobei
R³ = H, C₁- bis C₆-Alkyl oder -Cycloalkyl oder C₆- bis C₁₄-Aryl;
- Y =: O, S oder NR⁴
wobei
R⁴ = H, C₁- bis C₆-Alkyl oder -Cycloalkyl oder C₆- bis C₁₄-Aryl; und
- n =: 2 bis 6.

Die angegebenen Alkyl- und Alkylengruppen können linear, verzweigt oder cyclisch sein.

Die bei dem Rest R¹ gegebenenfalls vorhandenen Substituenten sind OH, Halogen, C₁- bis C₆-Alkoxy oder COOH, wobei es ebenfalls möglich ist, daß R¹ mehrfach substituiert ist. Sind mehrere Substituenten vorhanden, so können diese unabhängig voneinander gewählt werden.

Der Index n bei der allgemeinen Formel (I) bedeutet, daß die Gruppe R² n-fach mit dem in der Klammer angegebenen substituierten Vincylcyclopropan-Rest substituiert ist.

Weiter existieren für die oben angegebenen Variablen der Formel (I) unabhängig voneinander wählbare bevorzugte Definitionen, und diese sind wie folgt:
- R¹ =: H, ein- oder mehrfach mit OH, Halegen, C₁- bis C₆-Alkoxy, oder COOH substituiertes oder unsubstituiertes C₁- bis C₆-Alkyl oder Cycloalkyl oder C₆-Aryl;
- R² =: n-fach substituiertes C₁- bis C₁₂-Alkylen, oder -Cycloalkylen insbesondere C₆- bis C₈-Cycloalkylen, oder C₆- bis C₁₄- Arylen;
- X =: CO-O;
- R³ =: H oder C₁- bis C₃-Alkyl;
- Y =: O;
- R⁴ =: H oder C₁- bis C₃-Alkyl; und/oder
- n =: 2 oder 3.

Bevorzugte Verbindungen sind demgemäß solche, bei denen mindestens eine der Variablen der Formel (I) die vorstehend beschriebene bevorzugte Definition aufweist.

Üblicherweise liegen die erfindungsgemäßen Vinylcyclopropan-Derivate als Stereoisomeren-Gemischen und insbesondere als Racemate vor.

Zur Herstellung der erfindungsgemäßen multifunktionellen Vinylcyclopropan-Derivate wird in der Weise vorgegangen, daß man
ein Monovinylcyclopropan-Derivat der Formel (IIA) 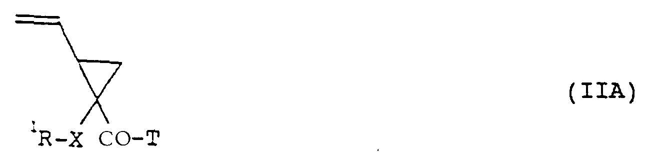 mit einer n-fach funktionalisierten Kupplungskomponente der Formel (IIB)

ZₙR² (IIB)

unter Bildung der multifunktionellen Vinylcyclopropan-Derivate der Formel (I) umsetzt, wobei
- T =: OH oder Halogen und
- Z =: OH, NH₂ oder Halogen
und die übrigen Variablen so wie vorstehend definiert sind.

Das erfindungsgemäße Herstellungsverfahren läßt sich demgemäß anhand der nachstehenden Reaktionsgleichung darstellen.

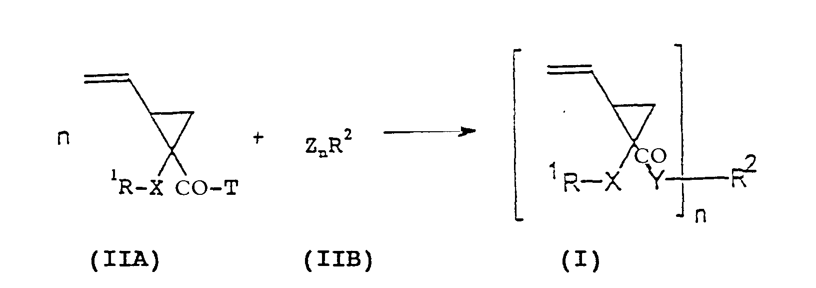

Dabei lassen sich Verbindungen der Formel (IIA) nach dem für die Herstellung von Vinylcyclopropanen bekannten Verfahren (vgl. US-A-4,713,478 und US-A-4,713,479) der Umsetzung von trans-1,4-Dihalogenbut-2-enen mit entsprechenden Malonsäurederivaten herstellen, wobei gegebenenfalls mit Schutzgruppen versehene Edukte eingesetzt werden müssen. Dieses Verfahren wird anhand eines Beispieles durch nachstehende Gleichungen illustriert. 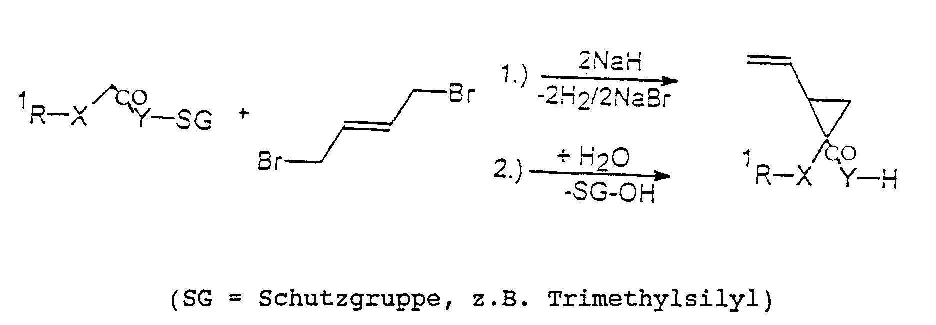 und gegebenenfalls

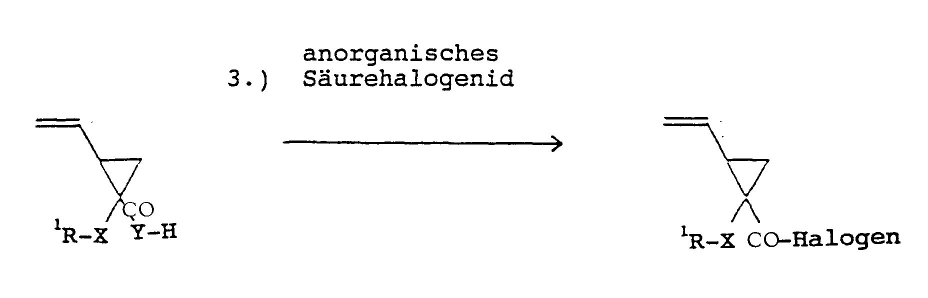

Mit dem erfindungsgemäßen Verfahren kann z.B. das difunktionelle Vinylcyclopropan (**4**) durch Veresterung von Resorcin mit 1-Methoxycarbonyl-1-chlorformyl-2-vinylcyclopropan, entsprechend nachstehender Reaktionsgleichung, erhalten werden:

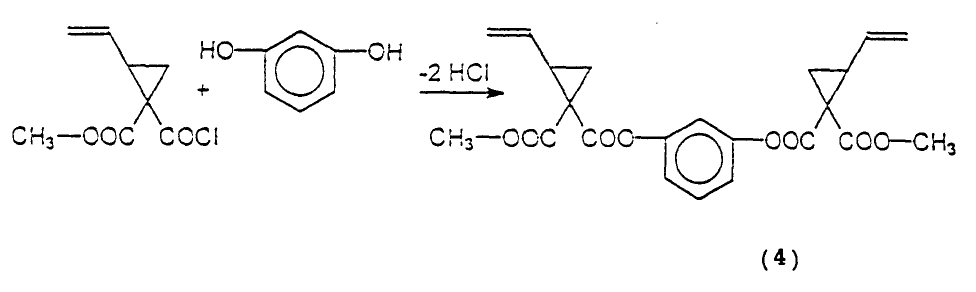

Mit dem erfindungsgemäßen Verfahren kann weiter z.B. das difunktionelle Vinylcyclopropan (3) durch Veresterung von Ethylenglycol mit 2-Vinylcyclopropan-1,1-dicarbonsäuremonomethylester, der seinerseits durch Umsetzung von käuflichem Malonsäuredimethylester mit trans-1,4-Dibrombut-2-en und nachfolgender partieller Verseifung des gebildeten 2-Vinylcyclopropan-1,1-dicarbonsäuredimethylesters zugänglich ist, entsprechend nachstehender Reaktionsgleichung erhalten werden:

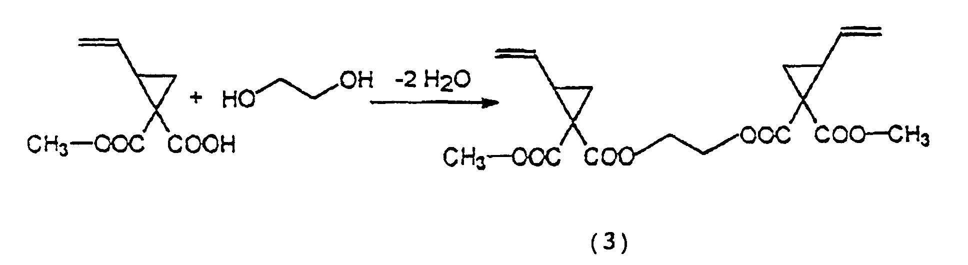

Als Kupplungskomponente (IIB) können insbesondere zwei- oder mehrwertige Hydroxyverbindungen, wie Ethylenglycol, Di- oder Triethylenglycol, Butylenglycol, 1,6-Hexandiol, Glycerin, Triethanolamin, Trimethylolpropantriol, Pentaerythrit oder Glucose, sowie Hydrochinon, Resorcin, Brenzkatechin oder Pyrogallol eingesetzt werden.

Darüber hinaus eignen sich auch di- oder multifunktionelle Amine, wie Ethylendiamin, Propylendiamin, Hexamethylendiamin sowie o-, p- oder m-Phenylendiamin.

Ferner können als Kupplungskomponente (IIB) auch insbesondere di- oder multifunktionelle organische Halogenverbindungen, wie z.B. 1,2-Dibromethan, 1,4-Dibrombutan, 1,10-Dibromdecan, 1,2,3-Tribrompropan, 1,4-Dibrombenzol oder 1,3,5-Tribrombenzol, eingesetzt werden. Diese Halogenverbindungen können z.B. mit dem Natriumsalz des Vinylcyclopropan-1,1-dicarbonsäuremonomethylesters zu dem entsprechenden erfindungsgemäßen Vinylcyclopropan-Derivat gemäß nachstehender Reaktionsgleichung umgesetzt werden.

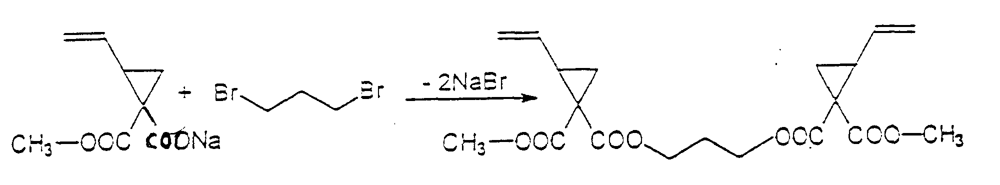

Neben dem zuvor beschriebenen Verfahren ist es auch möglich, daß zunächst durch Umsetzung der Verbindung (IIIA) mit n-fach funktioneller Kupplungskomponente (IIB) eine Zwischenstufe (IIIB) hergestellt wird, die dann durch Reaktion mit 1,4-Dibrombut-2-en in ein erfindungsgemäßes n-fach funktionelles Vinylcyclopropan-Derivat (I) gemäß folgender Reaktionsgleichungen überführt wird:

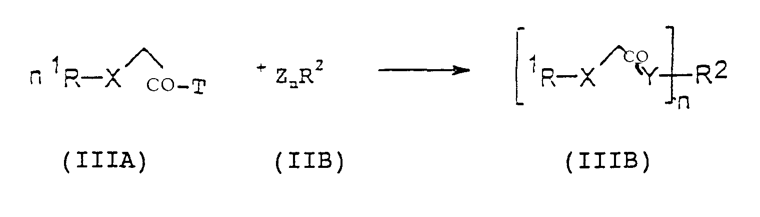

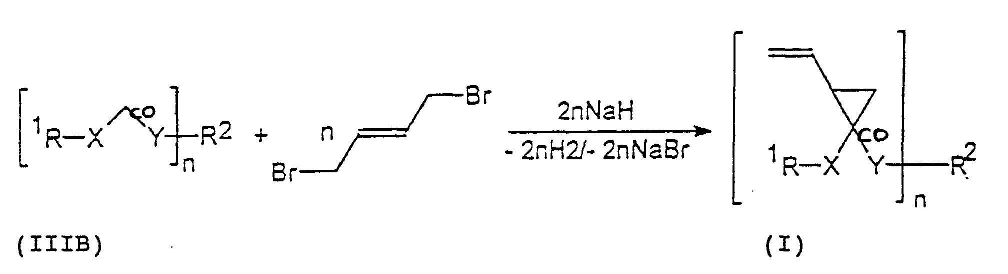

Bevorzugte Beispiele für die Verbindung (IIIA) sind dabei Malonsäurederivate.

Auf diese Weise kann z.B. das difunktionelle Vinylcyclopropan (4) dadurch hergestellt werden, daß man zunächst Resorcin mit Malonsäuremonomethylesterchlorid verestert und anschließend den gebildeten Ester mit 1,4-Dibrombut-2-en umsetzt, wie nachstehende Reaktionsgleichungen zeigen:

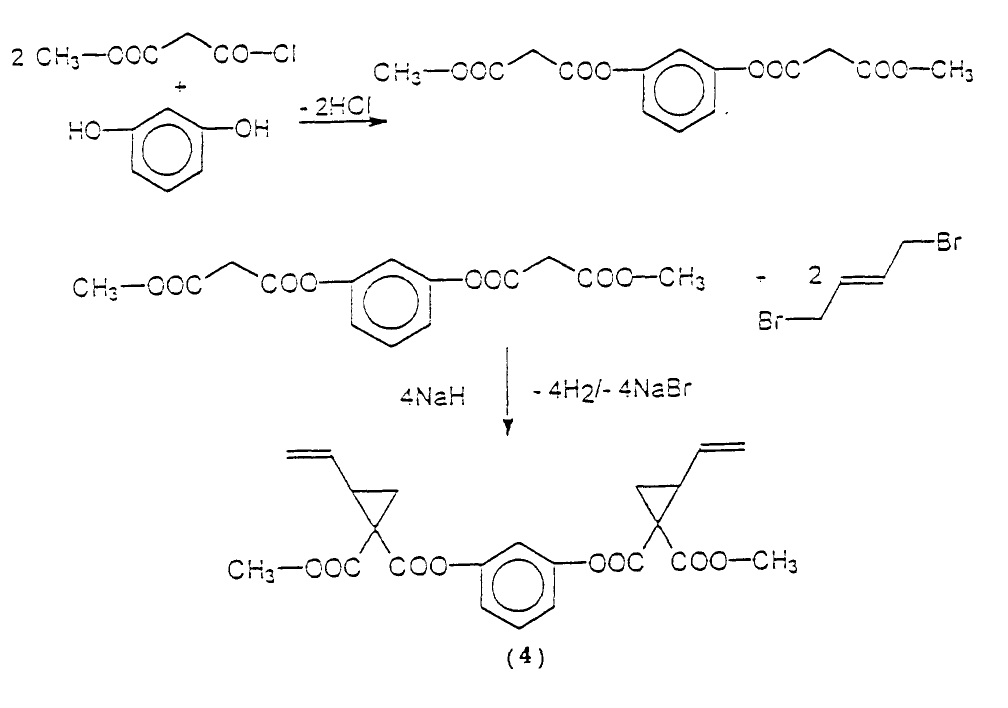

Aufgrund des Vorliegens von polymerisierbaren Gruppen eignen sich die erfindungsgemäßen multifunktionellen Vinylcyclopropan-Derivate als Ausgangsmaterialien für die Herstellung von Polymeren und Copolymeren. Dabei lassen sie sich mit den bekannten Methoden der radikalischen Polymerisation homopolymerisieren oder z.B. mit geeigneten Comonomeren copolymerisieren. Dabei erfolgt bei Verwendung herkömmlicher radikalischer Initiatoren weitestgehend eine Bildung von Polymeren und Copolymeren mit ringgeöffneten 1,5-Strukturen, was häufig zu einer Verringerung des Polymerisationsschrumpfes führt.

Die Durchführung der radikalischen Polymerisation erfolgt mit den bekannten radikalischen Initiatoren (vgl. Encyclopedia of Polymer Science and Engineering, Vol. 13, Wiley-Interscience Publisher, New York 1988, Seiten 754 ff.). Insbesondere eignen sich hierfür Azoverbindungen, wie Azobis(isobutyronitril) (AIBN) oder Azobis-(4-cyan-valeriansäure), oder Peroxide, wie Dibenzoylperoxid, Dilauroylperoxid, tert.-Butylperoctoat, tert.-Butylperbenzoat oder Di-(tert.-butyl)-peroxid.

Als Initiatoren für die Heißhärtung eignen sich vor allem Benzpinakol und 2,2'-Dialkylbenzpinakole.

Weiter können auch Photoinitiatoren (vgl. J.P. Fouassier, J.F. Rabek (Herausgeber), Radiation Curing in Polymer Science and Technology, Vol. II, Elsevier Applied Science, London und New York 1993) für die Polymerisation mit UV-Licht oder Licht sichtbarer Wellenlängen, wie Benzoinether, Dialkylbenzilketale, Dialkoxyacetophenone, Acylphosphinoxide, α-Diketone, wie 9,10-Phenanthrenchinon, Diacetyl, Furil, Anisil, 4,4'-Dichlorbenzil und 4,4'-Dialkoxybenzil und Campherchinon verwendet werden.

Zur Beschleunigung der Initiierung durch Peroxide oder α-Diketone können insbesondere Kombinationen mit aromatischen Aminen eingesetzt werden. Als Beschleuniger sind zudem Redoxsysteme einsetzbar, wie z.B. Kombinationen aus Benzoylperoxid, Lauroylperoxid oder Campherchinon mit Aminen, wie N,N-Dimethyl-p-toluidin, N,N-Dihydroxyethyl-p-toluidin oder strukturverwandten Aminen.

Die Vinylcyclopropane können insbesondere als Bestandteil von Adhäsiven, Zementen, Kompositen und Formkörpern sowie bevorzugt von Dentalmaterialien verwendet werden. Dabei ist es möglich, daß die Vinylcyclopropane in zumindest teilweise polymerisierter Form vorliegen. Weitere Komponenten, mit denen die Vinylcyclopropane kombiniert werden können, sind nachstehend erwähnt.

Die erfindungsgemäßen Vinylcyclopropan-Derivate lassen sich allein oder in Mischung mit herkömmlichen radikalisch polymerisierbaren Comonomeren, insbesondere mit difunktionellen Vernetzermonomeren, polymerisieren. Für die Herstellung von Adhäsiven oder Dentalmaterialien sind besonders geeignete Comonomere vor allem vernetzende bi- oder mehrfunktionelle Acrylate oder Methacrylate, wie z.B Bisphenol-A-di(meth)-acrylat, Additionsprodukte von Methacrylsäure und Bisphenol-A-diglycidylether, Urethandimethacrylate, z.B. das Additionsprodukt aus Hydroxyethylmethacrylat und 2,2,4-Trimethylhexamethylendiisocyanat, Di-, Tri- oder Tetraethylenglycoldi(meth)acrylat, Decandioldi(meth)acrylat, Trimethylolpropantri(meth)acrylat, Pentaerythrittetra(meth)acrylat, Butandioldi(meth)acrylat, 1,10-Decandioldi(meth)acrylat und 1,12-Dodecandioldi(meth)acrylat.

Darüber hinaus können die erfindungsgemäßen Vinylcyclopropan-Derivate oder ihre Mischungen mit anderen Monomeren zur Verbesserung der mechanischen Eigenschaften mit organischen oder anorganischen Partikeln oder Fasern gefüllt werden. Bevorzugte anorganische partikuläre Füllstoffe sind amorphe kugelförmige Materialien auf der Basis von Mischoxiden aus SiO₂, ZrO₂ und/oder TiO₂, mikrofeine Füllstoffe, wie pyrogene Kieselsäure oder Fällungskieselsäure, sowie Makro- oder Minifüllstoffe, wie Quarz-, Glaskeramik- oder Glaspulver mit einer durchschnittlichen Teilchengröße von 0,01 bis 5 µm sowie röntgenopake Füllstoffe, wie Ytterbiumtrifluorid. Darüber hinaus können auch Glasfasern, Polyamid- oder Kohlenstoff-Fasern eingesetzt werden.

Schließlich können den erfindungsgemäßen Vinylcyclopropan-Derivaten auch weitere Komponenten zugesetzt werden, wie z.B. Stabilisatoren, UV-Absorber, Farbstoffe, Pigmente oder Gleitmittel.

Die erfindungsgemäßen multifunktionellen Vinylcyclopropan-Derivate eignen sich besonders als Bestandteil von Dentalmaterialien, z.B. von Dentaladhäsiven, Befestigungszementen oder Füllungskompositen sowie von Materialien für Inlays/Onlays oder Zähne oder von Verblendmaterialien für Kronen und Brücken. Solche erfindungsgemäßen Dentalmaterialien zeichnen sich durch einen geringen Polymerisationsschrumpf und gute mechanische Eigenschaften aus. Sie können insbesondere auch bei Patienten mit (Meth)acrylat-Allergie eingesetzt werden. Da die polymerisierten erfindungsgemäßen Vinylcyclopropane darüber hinaus keine hydrolytisch spaltbaren Gruppen in der Hauptkette aufweisen, erweisen sie sich sich auch bei längerem Verbleib in der Mundhöhle als stabil, was bei Einsatz als Dentalmaterial naturgemäß von besonderem Vorteil ist.

Bevorzugte erfindungsgemäße Dentalmaterialien enthalten die folgenden Komponenten (a), (b), (c) und/oder (d):
(a) bis zu 99 Gew.-%, bevorzugt 10 bis 80 Gew.-% und besonders bevorzugt 20 bis 70 Gew.-% an den erfindungsgemäßen multifunktionellen Vinylcyclopropan-Derivaten,
(b) 0,01 bis 5 Gew.-%, besonders bevorzugt 0,1 bis 2,0 Gew.-% an Initiator,
(c) 0 bis 80 Gew.-%, bevorzugt 0 bis 60 Gew.-% und besonders bevorzugt 0 bis 50 Gew.-% an radikalisch polymerisierbaren Comonomeren,
(d) 0 bis 90 Gew.-%, besonders bevorzugt in Abhängigkeit von der Anwendung 0 bis 20 Gew.-% bei Adhäsiven, 20 bis 60 Gew.-% bei Zementen und 60 bis 85 Gew.-% bei Füllungskompositen an Füllstoffen.

Die Erfindung wird im folgenden anhand von Beispielen näher erläutert.

### Beispiele

### Beispiel 1: Synthese von 2-Vinylcyclopropan-1,1-dicarbonsäuremonomethylester (1)

In einem 100 ml Zweihalskolben mit Thermometer, Magnetrührer und CaCl₂-Rohr werden 36,8 g (0,2 Mol) 2-Vinylcyclopropan-1,1-dicarbonsäuredimethylester, der aus Malonsäuredimethylester und trans-1,4-Dibrombut-2-en zugänglich ist (vgl. US-A-4,713,478 und US-A- 4,713,479), in 65 ml Methanol gelöst, und die Lösung wird mit Eis-Wasser auf ca. 5°C gekühlt. Dann gibt man portionsweise 13,3 g (0,2 Mol) KOH so zu, daß die Temperatur nicht über 15°C steigt. Zur Vervollständigung der Reaktion wird 12 Stunden lang nachgerührt und dann am Rotationsverdampfer im Vakuum (50 mbar) bei 50°C eingeengt. Das erhaltene Öl (ca. 43 g) wird in 50 ml Wasser gelöst und unter Kühlung mit konzentrierter Salzsäure auf pH ca. 2-3 eingestellt. Man nimmt die organische Phase in 100 ml Diethylether auf, extrahiert noch zweimal mit je 100 ml Diethylether und trocknet die vereinigten Etherphasen über wasserfreiem Na₂SO₄. Die Lösung wird mit 0,01 g Hydrochinonmonomethylether stabilisiert, im Vakuum eingeengt und im Feinvakuum getrocknet. Es ergeben sich 28,2 g (83 % Ausbeute) einer farblosen Flüssigkeit.

| | | | | |
|---|---|---|---|---|
| Elementaranalyse | C₈H₁₀O₄ | Ber. | C 56,47 | H 5,92 |
| | (170,17) | Gef. | C 56,60 | H 5,82 |

- IR (Film, cm⁻¹):: 665 (w), 729 (w), 769 (w), 806 (w), 838 (w), 863 (w), 921 (m, sh), 958 (w), 992 (w), 1145 (s), 1209 (s, sh), 1289 (m), 1333 (s, sh), 1440 (s, sh), 1737 (s, sh, sh), 2957 (m), 3018 (m, b, sh).
- ¹H-NMR (90 MHz, CDCl₃):: 1,93 und 2,03 (s, 2× 1H, CH2-Cyclopropan); 2,88-2,90 (q, 1H, CH-Cyclopropan); 3,86 (s, 3H, CH₃); 5,18-5,69 (m, 3H, CH=CH₂); 12,30 (s, 1H, COOH, H/D-Austausch).

### Beispiel 2: Synthese von 1,1,1-Tris[(2-vinylcyclopropan-1-carbonsäuremethylester-1-carbonyloxy)methyl]propan (2)

In einem 100 ml Zweihalskolben mit Thermomether und CaCl₂-Rohr werden 9,45 g (55,6 mMol) des in Beispiel 1 hergestellten Esters (1), 2,5 g (18,7 mMol) 1,1,1-Tris(hydroxymethyl)propan, 0,061 g (0,5 mMol) 4-Dimethylaminopyridin (DMAP) in 40 ml absolutem Methylenchlorid gelöst, und die Lösung wird mit Eis-Wasser auf 0 bis 5 °C gekühlt. Unter Rühren und weiterem Kühlen werden portionsweise 11,45 g (55,6 mMol) N,N'-Dicyclohexylcarbodiimid (DCC) über einen Zeitraum von 1,5 Stunden zugegeben. Zur Vervollständigung der Reaktion rührt man noch 8 Stunden lang nach. Der ausgefallene Niederschlag wird abgesaugt, mit etwas Methylenchlorid gewaschen, und die vereinigten organischen Phasen werden nacheinander mit 100 ml 0,5N HCl, 60 ml gesättigter Natriumbicarbonatlösung und 50 ml gesättigter Kochsalzlösung extrahiert. Nach dem Abdestillieren des Methylenchlorids am Rotationsverdampfer bei 30 bis 40°C erhält man ein farbloses Öl, das zur Abtrennung von restlichem N,N'-Dicyclohexylharnstoff in 10 ml Aceton gelöst und nach Filtrieren wieder eingeengt wird. Nach weiterer Trocknung im Feinvakuum erhält man 7,1 g (64 % Ausbeute) eines klaren, farblosen, hochviskosen Öls.

| | | | | |
|---|---|---|---|---|
| Elementaranalyse | C₃₀H₃₈O₁₂ | Ber. | C 61,01 | H 6,48 |
| | (590,62) | Gef. | C 61,02 | H 6,97 |

- IR (Film, cm⁻¹):: 786 (w), 917 (m, sh), 992 (m), 1060 (w), 1130 (s), 1208 (s), 1268 (s), 1330 (s, sh), 1438 (m), 1520 (w), 1650 (w, sh), 1732 (s), 2954 (m, sh)
- ¹H-NMR (90 MHz, CDCl₃):: 0,90 (t, 3H CH₃CH₂); 1,30-1,85 (m, 8H, CH₂-Cyclopropan+CH₂CH₃); 2,55-2,75 (q, 3H, CH-Cyclopropan); 3,77 (s, 9H, CH₃O); 3,93-4,31 (q, 6H, CH₂O); 5,11-5,58 (m, 9H, CH=CH₂).

### Beispiel 3: Synthese von 1,2-Bis(2-vinylcyclopropan-1-carbonsäuremethylester-1-carbonyloxy)ethan (3)

Man arbeitet analog zu Beispiel 2 und setzt eine Lösung von 6,8 g (0,04 Mol) Ester (1), 1,25 g (0,02 Mol) absolutem Ethylenglykol und 0,061 g (0,5 mMol) DMAP in 20 ml Methylenchlorid mit 8,25 g (0,04 Mol) DCC um. Das erhaltene Öl wird im Feinvakuum unter Zugabe von etwas Phenothiazin destilliert, woraufhin 4,1 g (56% Ausbeute) eines farblosen Öls (Kp_{0,01 mbar} = 155 bis 160°C) erhalten werden.

| | | | | |
|---|---|---|---|---|
| Elementaranalyse | C₁₈H₂₂O₈ | Ber. | C 59,01 | H 6,05 |
| | (366,37) | Gef. | C 59,30 | H 6,30 |

- IR (Film, cm⁻¹):: 786 (w), 920 (w), 1132 (s), 1208 (s), 1270 (s), 1332 (s, sh), 1439 (m), 1645 (w), 1732 (s), 2956 (w, sh).
- ¹H-NMR (90 MHz, CDCl₃):: 1,52-1,83 (m, 2×2H, CH₂-Cyclopropan); 2,48-2,80 (q, 2×1H, CH-Cyclopropan); 3,75 (s, 2×3H, CH₃); 4,40 (s, 4H, OCH₂CH₂O); 5,12-5,60 (m, 2×3H, CH=CH₂).

### Beispiel 4: Synthese von Bis(2-vinylcyclopropan-1,1-dicarbonsäuremethyl)resorcinylester (4) - (Synthesevariante A)

Man arbeitet analog zu Beispiel 2 und setzt eine Lösung von 17,0 g (0,1 Mol) Ester (1), 5,5 g (0,02 Mol) Resorcin und 0,3 g (2,5 mMol) DMAP in 60 ml Methylenchlorid mit 20,6 g (0,1 Mol) DCC um. Das nach dem Abdestillieren des Methylenchlorids erhaltene Öl wird im Feinvakuum getrocknet. Nach einigen Tagen Stehen wird das Produkt (15,2 g, 73 % Ausbeute) fest, wobei die Differentialscanningkalorimetrie (DSC) einen Schmelzbereich von 30-80°C ergibt.

| | | | | |
|---|---|---|---|---|
| Elementaranalyse | C₂₂H₂₂O₈ | Ber. | C 63,76 | H 5,35 |
| | (414,41) | Gef. | C 63,51 | H 5,55 |

- IR (Film, cm⁻¹):: 460 (w), 677 (m), 714 (w), 792 (m, sh), 829 (w), 918 (s), 962 (m), 997 (m), 1026 (w), 1054 (m), 1131 (s), 1202 (s, sh), 1260 (s, sh), 1324 (s, sh), 1440 (s), 1485 (m), 1600 (m), 1638 (m), 1730 (s), 1755 (s), 2955 (m, sh), 3011 (w), 3090 (w), 3460 (w, b).
- ¹H-NMR (90 MHz, CDCl₃):: 1,65-2,03 (m, 4H, CH₂-Cyclopropan); 2,60-2,85 (q, 2H, CH-Cylcopropan); 3,82 (s, 6H, CH₃); 5,18-5,69 (m, 6H, CH=CH₂); 7,00-7,55 (m, 4H, CH-aromatisch).

### Beispiel 5: Synthese von Bis(2-vinylcyclopropan-1,1-dicarbonsäuremethyl)resorcinylester (4) - (Synthesevariante B)

### 1. Stufe: Bis(malonsäuremethyl)resorcinylester

In einem 750 ml Sulfierkolben mit mechanischem Rührwerk, Thermometer, Calciumchloridrohr und Tropftrichter legt man eine Lösung von 22,2 g (0,2 Mol) Resorcin, 44,5 g (0,44 Mol) Triethylamin und 2,48 g (0,02 Mol) DMAP in 200 ml absolutem THF vor und tropft unter gutem Rühren bei 0 bis 5°C innerhalb 1 Stunde eine Lösung von 74,4 g (0,53 Mol) Malonsäuremonomethylesterchlorid in 200 ml THF zu. Man läßt auf Raumtemperatur erwärmen, rührt das Reaktionsgemisch noch 5 Stunden lang nach, saugt das ausgefallene Triethylaminhydrochlorid ab, wäscht mit 200 ml Ether und extrahiert die vereinigten organischen Phasen 5× mit 100 ml 1N HCl, 8× mit 100 ml 10 %iger Na₂CO₃-Lösung und 5× mit 100 ml gesättigter NaCl-Lösung bis zu einer neutralen Reaktion. Es wird dann über wasserfreiem Na₂SO₄ getrocknet, am Rotationsverdampfer eingeengt und im Feinvakuum bis zur Gewichtskonstanz getrocknet. Das erhaltene Rohprodukt (34,5 g) wird säulenchromatographisch gereinigt, wobei 9 g (Ausbeute 15 %) reines Produkt erhalten werden.

| | | | | |
|---|---|---|---|---|
| Elementaranalyse | C₁₄H₁₄O₈ | Ber. | C 54,20 | H 4,55 |
| | (310,26) | Gef. | C 54,64 | H 4,47 |

- ¹H-NMR (90MHz, CDCl₃):: 3,60 (s, 4H, CH₂); 3,77 (s, 6H, CH₃); 7,0-7,5 (m, 4H, CH-aromatisch).

### 2. Stufe: Bis(2-vinyl-cyclopropandicarbonsäuremethyl)resorcinylester (4)

In einem 250 ml Dreihalsrundkolben mit Rückflußkühler und Thermometer werden 12,44 g (30,5 mMol) Natriumhydrid als 60 %ige Dispersion in Öl vorgelegt und unter Argon mit 50 ml absolutem Petrolether gewaschen. Der Petrolether wird dann abdekantiert. Man wiederholt diesen Vorgang zweimal und gibt schließlich 6,1 g (27,8 mMol) trans-1,4-Dibrom-2-buten und 90 ml absolutes THF zu. Danach tropft man unter Argon und bei Raumtemperatur innerhalb von 30 Minuten eine Lösung von 4,3 g (13,9 mMol) Bis(malonsäuremethyl) resorcinylester in 90 ml THF zu und rührt das erhaltene Reaktionsgemisch bei 65°C 4 Stunden lang. Dann engt man die erhaltene Suspension am Rotationsverdampfer bei 45°C zur Trockene ein, nimmt den Rückstand in 100 ml Diethylether auf und extrahiert 3× mit 100 ml gesättigter Na₂CO₃-Lösung und 4× mit 100 ml gesättigter NaCl-Lösung. Die erhaltene gelbe, klare Etherphase wird über wasserfreiem Na₂SO₄ getrocknet und am Rotationsverdampfer eingeengt. Der erhaltene Rückstand (4,2 g) wird mit je 50 ppm Di-tert. butyl-kresol und Phenothiazin stabilisiert, im Feinvakuum 24 Stunden lang getrocknet und mittels Flash-Chromatographie gereinigt. Es werden 2,4 g Produkt (43 % Ausbeute) erhalten, wobei das IR- und ¹H-NMR-Spektrum mit dem von Monomer (**4**) gemäß Beispiel 4 übereinstimmen.

### Beispiel 6: Radikalische Homopolymerisation von Vinylcyclopropan-Derivat (4)

1,005 g (2,4 mmol) Vinylcyclopropan-Derivat (**4**) werden mit 95 mg (0,06 mmol) Azobis(isobutyronitril) versetzt, im Schlenkgefäß entgast und anschließend bei 65°C unter Argon polymerisiert. Nach 15 Stunden wird die Polymerisation durch Abkühlen und Zugabe von 20 ml Chloroform abgebrochen. Aus der Dichtedifferenz von Monomer und unlöslichem Polymerisat (Gelanteil 19,5 %) konnte ein Polymerisationsschrumpf von lediglich ca. 9,5 Vol.% berechnet werden.

### Beispiel 7: Herstellung eines Dentalzementes auf der Basis von Vinylcyclopropan-Derivat (4)

Es wurden Kompositbefestigungszemente A) auf der Basis von einer konventionellen Methacrylatmischung, B) mit Gehalt an einem konventionellen monofunktionellen Vinylcyclopropan und C) mit Gehalt an dem erfindungsgemäßen difunktionellen Vinylcyclopropan-derivat (**4**) hergestellt. Die Zemente hatten die aus Tabelle 1 ersichtlichen Zusammensetzungen. Aus den Zementen wurden Prüfkörper hergestellt, die zweimal 3 Minuten lang mit einer dentalen Lichtquelle, nämlich Spectramat (Fa. Vivadent) bestrahlt wurden.

Aus Tabelle 2 ist erkennbar, daß das Material A mit der konventionellen Methacrylatmischung den größten Polymerisationsschrumpf aufweist. Das Material C ist dagegen sowohl in den mechanischen Eigenschaften als auch im Polymerisationsschrumpf den Materialien A und B überlegen.

**Tabelle 1:**

| Zementzusammensetzung | | | |
|---|---|---|---|
| **Komponenten** | **(A) Anteile (Gew.-%)** | **(B) Anteile (Gew.-%)** | **(C) Anteile (Gew.-%)** |
| Urethandimethacrylat¹⁾ | 31,6 | 31,6 | 31,6 |
| Dodecandioldimethacrylat | 7,80 | - | - |
| Mono(vinylcyclopropan)² | - | 7,80 | - |
| Bis(vinylcyclopropan) (**4**) | - | - | 7,80 |
| Aerosil OX-50³⁾ | 41,42 | 41,42 | 41,42 |
| Ytterbiumtrifluorid⁴⁾ | 18,70 | 18,70 | 18,70 |
| Campherchinon | 0,24 | 0,24 | 0,24 |
| N,N-Diethyl-3,5-di-tert.-butylanilin | 0,23 | 0,23 | 0,23 |
| 3,5-Di-tert.-butylkresol | 0,01 | 0,01 | 0,01 |

| | | | |
|---|---|---|---|
| ¹⁾ - Urethandimethacrylat aus 2 Mol 2-Hydroxyethylmethacrylat und 1 Mol 2,2,4-Trimethylhexamethylendiisocyanat-1,6. | | | |
| ²⁾ - 1,1-Bis(phenoxycarbonyl)-2-vinylcyclopropan, welches analog der Literaturvorschrift (vgl. J. Sugiama et al. Macromolecules **27**, (1994) 5543) hergestellt wurde. | | | |
| ³⁾ - Pyrogene Kieselsäure der Firma Degussa. | | | |
| ⁴⁾ - von der Firma Rhone-Poulenc. | | | |

**Tabelle 2:**

| Zementeigenschaften | | | |
|---|---|---|---|
| **Materialeigenschaft** | **A** | **B** | **C** |
| Polymerisationsschrumpfg (Vol.-%) | 4,9 | 2,0 | 1,0 |
| Biegefestigkeit nach ISO 4049 (MPa) | 86 | 49,5 | 88,5 |
| Biege-E-Modul nach ISO 4049 (GPa) | 3,19 | 1,73 | 4,44 |

## Patentansprüche

1. Multifunktionelle Vinylcyclopropan-Derivate der folgenden allgemeinen Formel (1), Stereoisomere davon und Gemische solcher Stereoisomeren 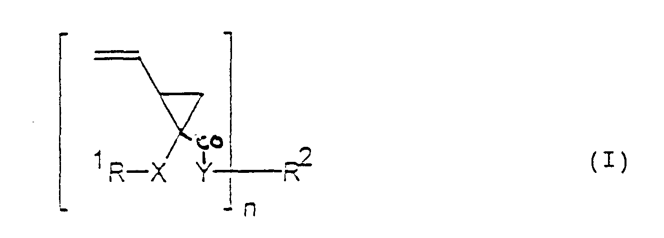 wobei R¹, R², R³, R⁴, X, Y und n unabhängig voneinander die folgenden Bedeutungen haben:
R¹ = H, ein- oder mehrfach mit OH, Halogen, C₁- bis C₆-Alkoxy oder COOH substituiertes oder unsubstituiertes C₁- bis C₁₂-Alkyl oder Cycloalkyl oder C₆- bis C₁₄-Aryl;
R² = n-fach substituiertes C₁- bis C₂₀-Alkylen oder - Cycloalkylen, das durch O, S, N oder NH unterbrochen sein kann, oder n-fach substituiertes C₆- bis C₁₄-Arylen;
X = CO, CO-O, CO-S, CO-NR³, SO₂ oder entfällt,
wobei
R³ = H, C₁- bis C₆-Alkyl oder -Cycloalkyl oder C₆- bis C₁₄-Aryl;
Y = O, S oder NR⁴
wobei
R⁴ = H, C₁- bis C₆-Alkyl oder -Cycloalkyl oder C₆- bis C₁₄-Aryl; und
n = 2 bis 6.

2. Vinylcyclopropan-Derivate nach Anspruch 1, **dadurch gekennzeichnet**, daß die Variablen der Formel (I) unabhängig voneinander die folgenden Bedeutungen haben:
R¹ = H, ein- oder mehrfach mit OH, Halogen, C₁- bis C₆-Alkoxy oder COOH substituiertes oder unsubstituiertes C₁- bis C₆-Alkyl oder -Cykloalkyl oder C₆-Aryl;
R² = n-fach substituiertes C₁- bis C₁₂-Alkylen oder - Cycloalkylen, insbesondere C₆- bis C₈-Cycloalkylen, oder C₆- bis C₁₄-Arylen;
X = CO-O;
R³ = H oder C₁- bis C₃-Alkyl oder -Cycloalkyl;
Y = O;
R⁴ = H oder C₁- bis C₃-Alkyl oder -Cycloalkyl; und/oder
n = 2 oder 3.

3. Verfahren zur Herstellung der Vinylcyclopropan-Derivate gemäß Anspruch 1 oder 2, **dadurch gekennzeichnet,** daß man
(A) ein Monovinylcyclopropan-Derivat der Formel (IIA) 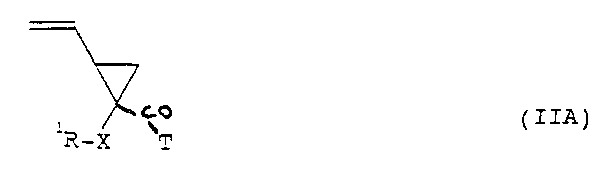 mit einer n-fach funktionalisierten Kupplungskomponente der Formel (IIB)
ZₙR² (IIB)
unter Bildung der multifunktionellen Vinylcyclopropan-Derivate der Formel (I) umsetzt,
oder
(B) eine Verbindung der Formel (IIIA) 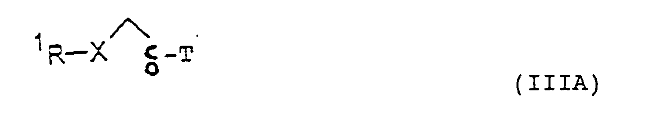 mit einer n-fach funktionalisierten Kupplungskomponente (IIB)
ZₙR² (IIB)
unter Bildung einer Zwischenstufe der Formel (IIIB) 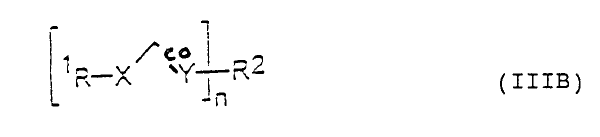 umsetzt und dann (IIIB) mit 1,4-Dibrombut-2-en unter Bildung der multifunktionellen Vinylcyclopropan-Derivate der Formel (I) zur Reaktion bringt,
wobei
T = OH oder Halogen und
Z = OH, NH₂ oder Halogen
und die übrigen Variablen so wie in Anspruch 1 definiert sind.

4. Verwendung der multifunktionellen Vinylcyclopropan-Derivate gemäß Anspruch 1 oder 2 als Bestandteil eines Adhäsivs, eines Zementes, eines Komposites, eines Formkörpers oder eines Dentalmaterials.

5. Verwendung nach Anspruch 4, **dadurch gekennzeichnet,** daß die Vinylcyclopropan-Derivate in zumindest teilweise polymerisierter Form vorliegen.

6. Dentalmaterial, **dadurch gekennzeichnet,** daß es ein multifunktionelles Vinylcyclopropan-Derivat gemäß Anspruch 1 oder 2 enthält.

7. Dentalmaterial nach Anspruch 6, **dadurch gekennzeichnet,** daß es das Vinylcyclopropan-Derivat in zumindest teilweise polymerisierter Form enthält.

8. Polymere und Copolymere, **dadurch gekennzeichnet,** daß sie durch Polymerisation oder Copolymerisation der mehrfunktionellen Vinylcyclopropan-Derivate gemäß Anspruch 1 oder 2 erhältlich sind.

## Claims

1. Multifunctional vinylcyclopropane derivatives of the following general formula (I), stereoisomers thereof and mixtures of such stereoisomers 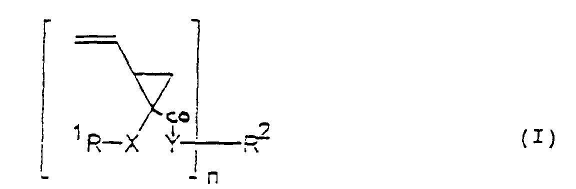 where R¹, R², R³, R⁴, X, Y and n independently of one another have the following meanings:
R¹ = H, unsubstituted or OH-, halogen-, C₁ to C₆ alkoxy- or COOH-monosubstituted or -polysubstituted C₁ to C₁₂ alkyl or cycloalkyl or C₆ to C₁₄ aryl;
R² = n-tuply substituted C₁ to C₂₀ alkylene or cycloalkylene which can be interrupted by O, S, N or NH, or n-tuply substituted C₆ to C₁₄ arylene;
X = CO, CO-O, CO-S, CO-NR³, SO₂ or is absent, where
R³ = H, C₁ to C₆ alkyl or cycloalkyl or C₆ to C₁₄ aryl;
Y = O, S or NR⁴,
where
R⁴ = H, C₁ to C₆ alkyl or cycloalkyl or C₆ to C₁₄ aryl; and
n = 2 to 6.

2. Vinylcyclopropane derivatives according to Claim 1, characterized in that the variables of the formula (I) independently of one another have the following meanings:
R¹ = H, unsubstituted or OH-, halogen-, C₁ to C₆ alkoxy- or COOH-monosubstituted or -polysubstituted C₁ to C₆ alkyl or cycloalkyl or C₆ aryl;
R² = n-tuply substituted C₁ to C₁₂ alkylene or cycloalkylene, in particular C₆ to C₈ cycloalkylene, or C₆ to C₁₄ arylene;
X = CO-O;
R³ = H or C₁ to C₃ alkyl or cycloalkyl;
Y = O;
R⁴ = H or C₁ to C₃ alkyl or cycloalkyl; and/or
n = 2 or 3.

3. Process for the preparation of the vinylcyclopropane derivatives according to Claim 1 or 2, characterized in that
(A) a monovinylcyclopropane derivative of the formula (IIA) 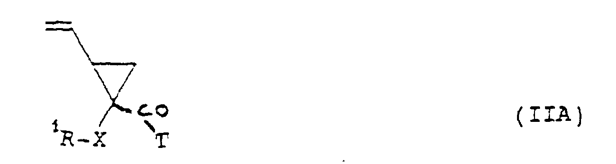 is reacted with an n-times functionalized coupling component of the formula (IIB)
ZₙR² (IIB)
with formation of the multifunctional vinylcyclopropane derivatives of the formula (I),
or
(B) a compound of the formula (IIIA) 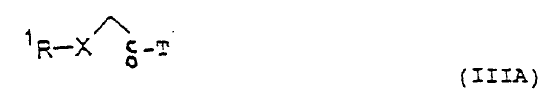 is reacted with an n-tuply functionalized coupling component (IIB)
ZₙR² (IIB)
with formation of an intermediate of the formula (IIIB) 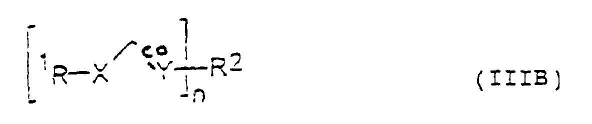 and then (IIIB) is reacted with 1,4-dibromobut-2-ene with formation of the multifunctional vinylcyclopropane derivatives of the formula (I),
where
T = OH or halogen and
Z = OH, NH₂ or halogen
and the remaining variables are as defined in Claim 1.

4. Use of the multifunctional vinylcyclopropane derivatives according to Claim 1 or 2 as a constituent of an adhesive, cement, composite or dental material.

5. Use according to Claim 4, characterized in that the vinylcyclopropane derivatives are present in at least partially polymerized form.

6. Dental material, characterized in that it contains a multifunctional vinylcyclopropane derivative according to Claim 1 or 2.

7. Dental material according to Claim 6, characterized in that it contains the vinylcyclopropane derivative in at least partially polymerized form.

8. Polymers and copolymers, characterized in that it can be obtained by polymerization or copolymerization of the multifunctional vinylcyclopropane derivatives according to Claim 1 or 2.

## Revendications

1. Dérivés multifonctionnels de vinylcyclopropane de formule générale (I), leurs stéréoisomères et mélanges de tels stéréoisomères 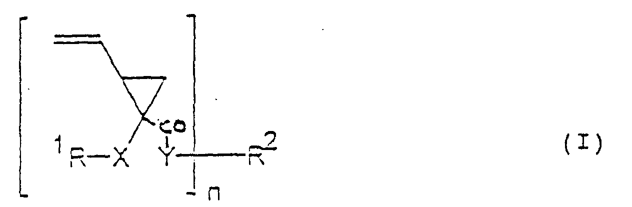 formule dans laquelle R¹, R², R³, R⁴, X, Y et n ont indépendamment les uns des autres les significations suivantes :
R¹ = H, un groupe alkyle ou cycloalkyle en C₁-C₁₂ ou aryle en C₆-C₁₄, non substitué ou substitué une ou plusieurs fois par un ou des atomes d'halogène ou groupes OH, alcoxy en C₁-C₆ ou COOH ;
R² = un groupe alkylène ou cycloalkylène en C₁-C₂₀ n fois substitué, qui peut être interrompu par O, S, N ou NH, ou un groupe arylène en C₆-C₁₄ n fois substitué ;
X = CO, CO-O, CO-S, CO-NR³, SO₂ ou est absent,
R³ étant H ou un groupe alkyle ou cycloalkyle en C₁-C₆ ou aryle en C₆-C₁₄;
Y = O, S ou NR⁴,
R⁴ représentant H ou un groupe alkyle ou cycloalkyle en C₁-C₆ ou aryle en C₆-C₁₄; et
n = 2 à 6.

2. Dérivés de vinylcyclopropane selon la revendication 1, caractérisés en ce que les variables de la formule (I) ont, indépendamment les unes des autres, les significations suivantes :
R¹ = H, un radical alkyle ou cycloalkyle en C₁-C₆ ou aryle en C₆, non substitué ou une ou plusieurs fois substitué par un ou des atomes d'halogène ou groupes OH, alcoxy en C₁-C₆ ou COOH ;
R² = un groupe alkylène ou cycloalkylène en C₁-C₁₂, en particulier un groupe cycloalkylène en C₆-C₈, ou un groupe arylène en C₆-C₁₄, n fois substitué ;
X = CO-O ;
R³ = H ou un groupe alkyle ou cycloalkyle en C₁-C₃ ;
Y = O;
R⁴ = H ou un groupe alkyle ou cycloalkyle en C₁-C₃; et/ou
n = 2 ou 3.

3. Procédé pour la préparation des dérivés de vinylcyclopropane selon la revendication 1 ou 2, caractérisé en ce que
(A) on fait réagir un dérivé de monovinylcyclopropane de formule (IIA) 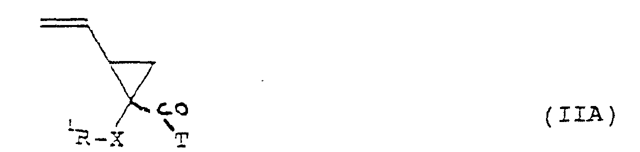 avec un composant de couplage n fois fonctionnalisé de formule (IIB)
ZₙR² (IIB)
avec formation des dérivés multifonctionnels de vinylcyclopropane de formule (I), ou
(B) on fait réagir un composé de formule (IIIA) 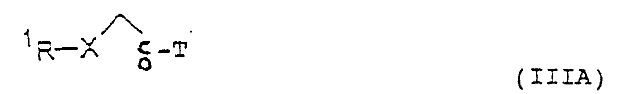 avec un composant de couplage n fois fonctionnalisé de formule (IIB)
ZₙR² (IIB)
avec formation d'un composé intermédiaire de formule (IIIB) 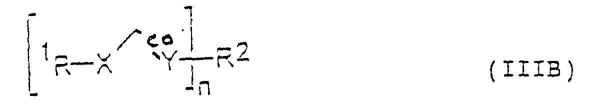 et on fait ensuite réagir (IIIB) avec du 1,4-dibromobut-2-ène, avec formation des dérivés multifonctionnels de vinylcyclopropane de formule (I),
T représentant OH ou un atome d'halogène et
Z représentant OH, NH₂ ou un atome d'halogène,
et les autres variables étant telles que définies dans la revendication 1.

4. Utilisation des dérivés multifonctionnels de vinylcyclopropane selon la revendication 1 ou 2, en tant que composant d'un adhésif, d'une colle, d'un composite, d'un corps moulé ou d'un matériau dentaire.

5. Utilisation selon la revendication 4, caractérisée en ce que les dérives de vinylcyclopropane se trouvent sous forme au moins en partie polymérisée.

6. Matériau dentaire, caractérisé en ce qu'il contient un dérivé multifonctionnel de vinylcyclopropane selon la revendication 1 ou 2.

7. Matériau dentaire selon la revendication 6, caractérisé en ce qu'il contient le dérivé de vinylcyclopropane sous forme au moins en partie polymérisée.

8. Polymères et copolymères, caractérisés en ce qu'ils peuvent être obtenus par polymérisation ou copolymérisation des dérivés multifonctionnels de vinylcyclopropane selon la revendication 1 ou 2.
